# EUROPEAN PATENT SPECIFICATION

(11) **EP 0 253 398 B1**
(45) Date of publication and mention of the grant of the patent: **10.03.1993**
(21) Application number: 87110314.9
(22) Date of filing: 16.07.1987
(51) Int. Cl.: A61N 2/12, H02K 21/00

(54) **Magnetic health device**
Magnetische Gesundheitseinrichtung
Dispositif de santé magnétique

(30) Priority: 18.07.1986 JP 109423/86
(43) Date of publication of application: 20.01.1988
(73) Proprietor: Ohama, Haruo, Naha-shi Okinawa-ken (JP)
(72) Inventor: Ohama, Haruo, Naha-shi Okinawa-ken (JP)
(74) Representative: Schmidt-Evers, Jürgen, Dipl.-Ing.

(56) References cited:
- EP-A- 0 084 019
- EP-A- 0 159 043
- AT-B- 11 766
- DE-A- 3 221 544
- DE-A- 3 417 773
- US-A- 4 029 980
- US-A- 4 482 829
- US-A- 4 537 181

## Description

This invention relates to a small-sized magnetic health device which can be used, for the purpose of keeping one's health, in such a manner that the same is carried with a user or attached to clothes, a belt, spectables or others of the user.

As for a conventional small-sized magnetic health device, there has been hitherto known only such a type one utilizing a stationary magnetic field of a magnet piece or the like for keeping one's health.

With the progress of researches on an influence of magnetism on a human body, there has been obtained such a finding that, rather than the stationary magnetic field, a movable magnetic field has a larger acceleration effect on circulation of body fluids such as blood in a human body. Accordingly, it has been desired to develop, on the basis of the foregoing finding, a new magnetic health device which utilizes the moveable magnetic field and is convenient in carrying or the like.

For meeting this desire, the applicant of this application has previously proposed such a magnetic health device that a magnetic rotary member which has mutually opposite magnetic poles in its turning direction and an electric motor for turning the same are housed in a casing. This type one, however, is inconvenient in that, since the same is provided with the motor housed therein, the same becomes comparatively large in the external dimensions and comparatively large in in weight, so that especially when the same is attached to a small article such as spectacles or the like, it gives a user a sense of incompatibility or an unpleasant feeling.

US-A-4 482 829 discloses a brushless electric micromotor comprising a rotor, a shaft and permanent magnets rotatably mounted in a rotor housing. The permanent magnets have their co-axial surfaces coated with a layer of an electrically conductive metal. The micromotor further comprises a stator co-axially surrounding the rotor housing and comprising field windings regularly arranged with equal angular distances in the stator housing made of magnetic material.
The object of the present invention is to provide a device having small external dimensions and in which the starting of the rotation is made smooth and the turning thereof is made stable.
This object is solved by the characterising features of claim 1.

Next, the operation of this invention device will be explained as follows:-
An electric current which is changed over between a regular direction and a reverse direction alternately in a constant cycle is flowed through the coil, and thereby the magnetic rotary member is given a rotating torque in one direction, and there is created a movable magnetic field such as a rotating magnetic field around the magnetic rotary member, so that if the device is brought to a desired part of a human body, circulation of a body fluid is accelerated.

Embodying examples of this invention will now be explained with reference to the accompanying drawings:-

### BRIEF EXPLANATION OF THE DRAWINGS

Fig. 1 is a sectional elevational view of one embodying example of this invention,
Fig. 2 is a top plan view of a rotary member unit thereof,
Fig. 3 is an elevational view of the same,
Fig. 4 is a side view of the same,
Fig. 5 is a sectional view taken along the line V - V in Fig. 2,
Fig. 6 is a sectional view taken along the line VI - VI in Fig. 2,
Fig. 7 is a circuit diagram of an electric source unit thereof,
Fig. 8 is a sectional view for explaining a stopped state of a magnetic rotary member thereof,
Fig. 9 is a perspective view of the magnetic rotary member,
Fig. 10 is a perspective view of a modified example thereof,
Fig. 11 is a perspective view of a modified embodying example of this invention,
Fig. 12 is a perspective view of another embodying example of this invention, and
Fig. 13 is a sectional view taken along the line XIII-XIII in Fig. 12.

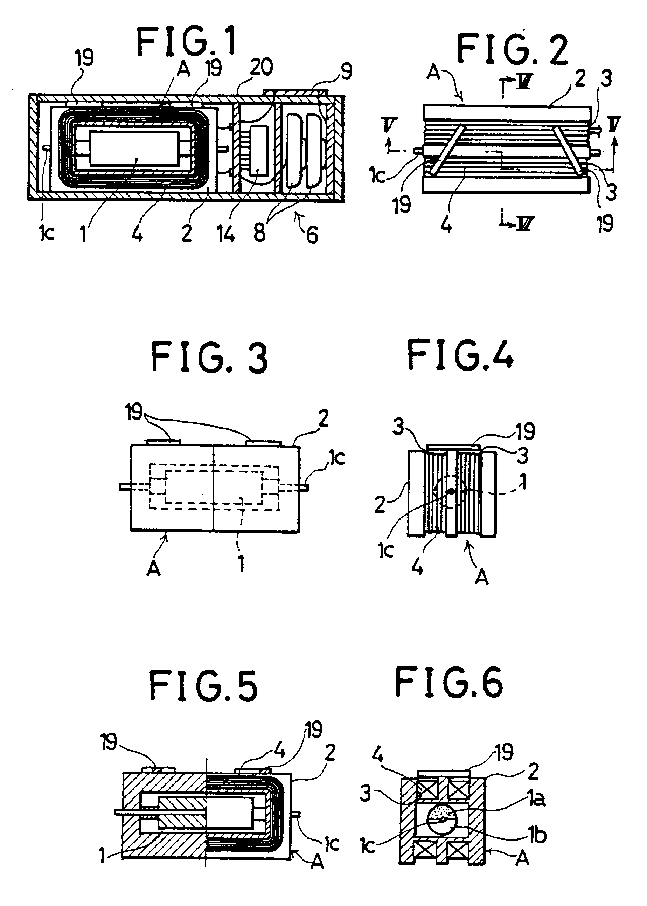

Referring to Figs. 1 - 9 showing one embodying example thereof, numeral 1 denotes a magnetic rotary member, and this rotary member 1 is provided with a coil 4 through a stationary frame 2, and thus there is constructed a rotary member unit A by these members.

More in detail, as shown clearly in Fig. 2 - 6, the unit A is so constructed that the foregoing magnetic rotary member 1 in a cylindrical form which comprises two divisions in its rotating direction and has such mutually opposite magnetic poles that one of the two divisions is an N pole 1a and the other thereof is an S pole 1b, is put in and turnably supported on the foregoing stationary frame 2 made of plastics, for instance, and the foregoing coil 4 is so formed along in two parallel grooves 3, 3 made in an outer circumferential surface of the stationary frame 2 that its axis may cross at right angle with the direction of a rotary shaft 1c of the magnetic rotary member 1, and both ends of the coil 4 are connected to external terminals 7, 7 of an electric source unit 6, through leading wires 5, 5.

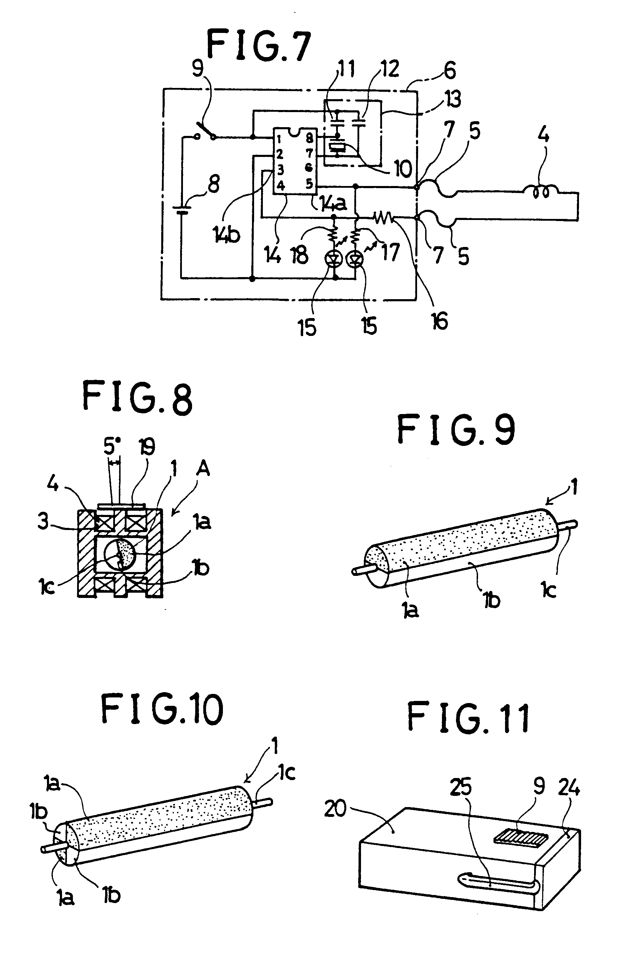

The electric source unit 6 comprises, as shown clearly in Fig. 7, a battery 8, a power switch 9, an oscillator 13 comprising a crystal oscillator 10 and two condensers 11, 12, an outputting IC element 14 which is so arranged that an output from the oscillator 13 is inputted thereto and thereby predetermined voltages are outputted from output terminals 14a, 14b thereof connected to the foregoing external terminals and light-emitting diodes 15, 15 to be made on and off by the output from the IC element 14.

Thus, if the power switch 9 is closed, the voltage of the battery 8 is applied to the oscillator 13 and the IC element 14, whereby pulse voltages of a constant or periodic cycle, for instance, those at a rate of one pulse per second, are outputted from the oscillator 13 and are inputted to the IC element 14, and thereby high level pulse voltages are outputted periodically in a constant cycle from the output terminal 14a and the output terminal 14b of the IC element 14, alternatively and at the same time the light-emitting diodes 15, 15 are made on and off periodically in the constant cycle.

As a result, the high level pulse voltages appearing at the output terminal 14a and at the output terminal 14b alternately are applied to the external terminals 7, 7, whereby an electric current which is changed over between that of a regular direction and that of the reverse direction at a constant period of time is flowed through the coil 4, and thereby the magnetic rotary member 1 is rotated and there is created a movable magnetic field around the magnetic rotary member 1.

Numerals 16, 17, 18 denote electric current limiting resistances.

The foregoing embodying example is of such an intermittent turning type that if one of the high level pulse voltages is outputted, the magnetic rotary member 1 is turned by about 180 degrees, and then the magnetic rotary member 1 is once stopped until the next one of the high level pulse voltages is outputted.

In this case, in order that the starting of the rotation thereof may be made smooth and the turning thereof may be made stable, it is desirable that the magnetic rotary member 1 is always once stopped at the same place.

For this purpose, the coil 4 is provided thereon with a pair of iron members 19, 19 inclined inwards one with another, as shown clearly in Fig. 2, so that the magnetic rotary member 1 can be stopped in such a condition that either one of the poles of the magnetic rotary member 1 is attracted to such end portions of the two iron members 19, 19 that are narrower in space distance therebetween toward ends thereof, that is, in such a condition that, as shown in Fig. 8, the boundary line between the two poles 1a, 1b is inclined by about 5 degrees in relation to the wound surface of the coil 4.

The magnetic rotary member 1 as shown in Fig. 9 may be so modified that, as shown in Fig. 10, the same comprises four divisions in its turning direction, and these four divisions are magnetically an N pole 1a, an S pole 1b, an N pole and an S pole alternately. Additionally, the magnetic rotary member 1 is not limited to be in a circular column form but may be in a square column form or the like.

Additionally, it is desirable to make the number of pulses per minute from the oscillator 13 equal to the number of heartbeat.

In this embodying example, as shown clearly in Fig. 1, the rotary member unit A and the electric source unit 6 are housed in a single common casing 20 so as to be portable, and, as an occasion demands, the same is applied to a desired part of a human body of a user, the health effect thereof can be obtained.

Fig. 11 shows a modified example thereof. In this example, a clip 25 is attached to a closure member 24 which detachably provided on an end portion of the casing 20 so as to replace the batteries 8 so that the casing 20 may be attached detachably, through the clip 25, to clothes, a belt or the like.

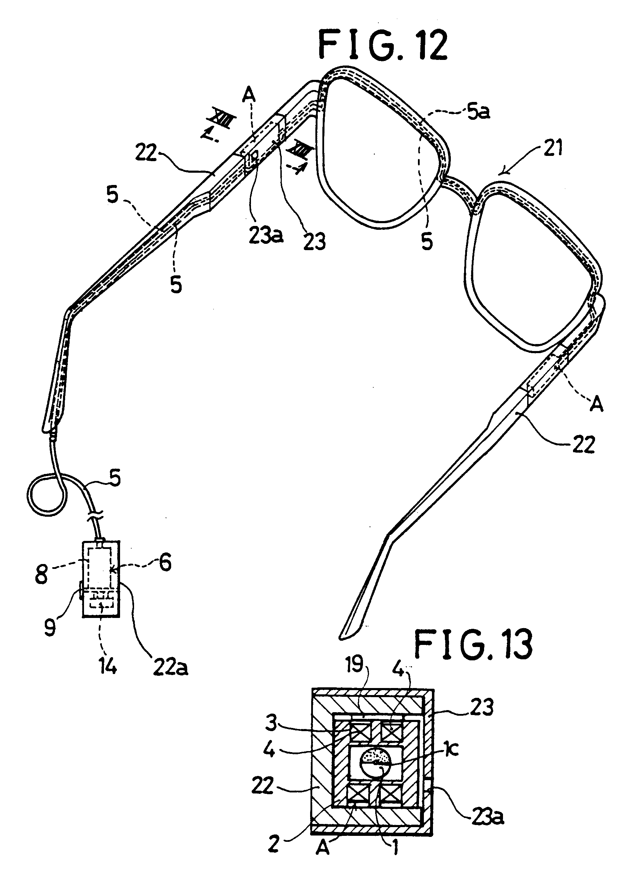

Figs. 12 and 13 show further another embodying example of this invention. In this example, the rotary member unit A and the electric source unit 6 are housed in individuable casing 22, 22a, and the casing 22 for the unit A is formed of a temple of each side of spectacles 21.

More in detail, a pair of the magnetic rotary units A, A are embedded in the temples 22, 22 of both sides of the spectacles 21, and the two coils 4, 4 of these units A, A are connected, in series, one with another through a leading wire 5a, and both ends of the wire 5a are led out to the exterior through the leading wires 5, 5, and the external outer ends thereof are connected to the external terminals 7, 7 of the electric source unit 6 housed in the foregoing portable casing 22a put in a pocket or the like.

In this example, the N pole and the S pole of the magnetic rotary member 1 are discriminated from each other in different colors so that the rotating state thereof may be observed through a window 23a made through a covering member 23 applied to a side surface thereof.

## Claims

1. A magnetic health device, comprising :
a magnetic rotary member (1) which has mutually opposite magnetic poles (1a, 1b) in its rotating direction, and a coil (4) which has its axis crossing at right angle with the direction of a rotary shaft (1c) of the magnetic rotary member (1) is so provided around the periphery of the magnetic rotary member (1) as to leave a space therebetween, whereby there is constructed a rotary member unit (A), and the coil (4) thereof is connected to an electric source unit (6) for supplying an electric current in such a manner that a flowing direction thereof is changed over between a regular direction and a reverse direction alternately in a constant cycle,
characterized in that
the coil (4) is provided thereon with a pair of iron members (19) so inclined one with another as to become narrower in a distance therebetween toward one end thereof so that the magnetic rotary member (1) may be stopped by attracting either one of the poles to such end portions of the two iron members (19) that are narrower in the distance therebetween.

2. A magnetic health device as claimed in claim 1 , characterized in that the magnetic rotary member (1) is formed of two divisions and one of the two divisions is magnetically an N pole, and the other thereof is magnetically an S pole.

3. A magnetic health device as claimed in claim 1, characterized in that the magnetic rotary member (1) is formed of four divisions, and these regions are so arranged as to be magnetically an N pole, an S pole, an N pole and an S pole, alternately.

4. A magnetic health device as claimed in any one of claims 1 to 3, characterized in that the rotary member unit (A) and the electric source unit (6) are housed in a single common casing (20).

5. A magnetic health device as claimed in claim 4, characterized in that the casing (20) is provided with a clip (25).

6. A magnetic health device as claimed in any one of claims 1 to 3, characterized in that the rotary member unit (A) is housed in a casing (22, 23), and the electric source unit (6) is housed in another casing (22a) provided outside the foregoing casing (22, 23).

7. A magnetic health device as claimed in claim 6, characterized in that the casing for containing the rotary member unit (A) is composed of a temple (22) of spectacles (21).

8. A magnetic health device as claimed in any one of claims 1 to 7, characterized in that the electric source unit (6) generates pulse voltages of a constant or periodic cycle.

## Patentansprüche

1. Magnetische Gesundheitseinrichtung mit einem magnetischen Rotationselement (1), welches einander entgegengesetzte Magnetpole (1a,1b) in seiner Rotationsrichtung aufweist, und einer Spule (4), die eine Achse hat, die rechtwinklig mit der Richtung der Drehwelle (1c) des magnetischen Rotationselements (1) sich kreuzt, die so am Umfang des magnetischen Rotationselements (1) angeordnet ist, daß dort eine Lücke dazwischen gelassen wird, wodurch eine Baugruppe (A) eines Rotationselements gebildet wird, dessen Spule (4) mit einer Baugruppe (6) einer elektrischen Spannungsquelle verbunden ist, die einen elektrischen Strom in einer Weise liefert, daß dessen Fließrichtung zwischen einer regulären Richtung und einer umgekehrten Richtung abwechselnd in einem konstanten Zyklus wechselt,
**dadurch gekennzeichnet,**
daß auf der Spule (4) zwei Eisenteile (19) vorgesehen sind, die so zueinander geneigt sind, daß an einem Ende ein kleinerer Abstand zwischen ihnen entsteht, so daß das magnetische Rotationselement (1) durch Anziehen einer der beiden Pole auf die Endbereiche der beiden Eisenteile (19) angehalten werden kann, die einen kleineren dazwischenliegenden Abstand haben.

2. Magnetische Gesundheitseinrichtung nach Anspruch 1, dadurch gekennzeichnet, daß das magnetische Rotationselement (1) durch zwei Teile gebildet wird, wobei einer der beiden Teile ein magnetischer Nordpol und der andere ein magnetischer Südpol ist.

3. Magnetische Gesundheitseinrichtung nach Anspruch 1, dadurch gekennzeichnet, daß das magnetische Rotationselement (1) durch vier Teile gebildet wird, wobei diese Bereiche so angeordnet sind, daß sie nacheinander einen magnetischen Nordpol, einen Südpol, einen Nordpol und einen Südpol bilden.

4. Magnetische Gesundheitseinrichtung nach einem der Ansprüche 1 bis 3, dadurch gekennzeichnet, daß die Baugruppe (A) des Rotationselements und die Baugruppe (6) der elektrischen Spannungsquelle in einem einzigen gemeinsamen Gehäuse (20) angeordnet sind.

5. Magnetische Gesundheitseinrichtung nach Anspruch 4, dadurch gekennzeichnet, daß das Gehäuse (20) mit einem Clip (25) versehen ist.

6. Magnetische Gesundheitseinrichtung nach einen der Ansprüche 1 bis 3, dadurch gekennzeichnet, daß die Baugruppe (A) des Rotationselements in einem Gehäuse (22, 23) untergebracht ist und die Baugruppe (6) der elektrischen Spannungsquelle in einem anderen Gehäuse (22a) untergebracht ist, das außerhalb des obigen Gehäuses (22, 23) vorgesehen ist.

7. Magnetische Gesundheitseinrichtung nach Anspruch 6, dadurch gekennzeichnet, daß das Gehäuse für die Baugruppe (A) des Rotationselements aus einem Bügel (22) einer Brille (21) besteht.

8. Magnetische Gesundheitseinrichtung nach einen der Ansprüche 1 bis 7, dadurch gekennzeichnet, daß die Baugruppe (6) der elektrischen Spannungsquelle Impulsspannungen mit einem konstanten oder periodischen Zyklus erzeugt.

## Revendications

1. Dispositif de stimulation physiologique magnétique comportant :
un élément rotatif magnétique (1) comprenant des pôles magnétiques mutuellement opposés (1a, 1b) dans le sens de sa rotation et un enroulement (4) dont l'axe est perpendiculaire à l'axe de l'arbre rotatif (1c) de l'élément rotatif magnétique (1) monté autour de la périphérie de l'élément rotatif magnétique (1) de manière à ménager un espace entre l'enroulement et l'élément rotatif magnétique, l'ensemble constituant une unité d'élément rotatif (A) ; l'enroulement (4) est connecté à une unité de source d'énergie électrique (6) qui fournit un courant électrique dont le sens d'écoulement varie alternativement dans un sens donné et dans un sens opposé suivant un cycle constant,
caractérisé en ce que
l'enroulement (4) comporte une paire de pièces en fer (19) inclinées l'une par rapport à l'autre de manière à ce que l'une de leurs extrémités respectives soient voisines l'une de l'autre, l'élément rotatif magnétique (1) pouvant de la sorte être arrêté par l'attraction exercée sur l'un des pôles par lesdites parties d'extrémité les plus voisines, des pièces en fer (19).

2. Dispositif de stimulation physiologique magnétique selon la revendication 1, caractérisé en ce que l'élément rotatif magnétique (1) est formé par deux sections, l'une d'entre elle constituant le pôle magnétique N, l'autre section constituant le pôle magnétique S.

3. Dispositif de stimulation physiologique magnétique selon la revendication 1, caractérisé en ce que l'élément rotatif magnétique (1) est formé par quatre sections, ces régions étant disposées alternativement de manière à constituer un pôle magnétique N, un pôle S, un pôle N et un pôle S.

4. Dispositif de stimulation physiologique magnétique selon l'une quelconque des revendications 1 à 3, caractérisé en ce que l'unité (A) d'élément rotatif et l'unité de source d'énergie électrique (6) sont logées dans un boîtier commun unique (20).

5. Dispositif de stimulation physiologique magnétique sel on la revendication 4, caractérisé en ce que le boîtier 20 comporte une boucle (25).

6. Dispositif de stimulation physiologique magnétique selon l'une quelconque des revendications 1 à 3, caractérisé en ce que l'unité d'élément rotatif (A) est logée dans un boîtier (22, 23) et en ce que l'unité de source d'énergie électrique (6) est logée dans un autre boîtier (22a) prévu à l'extérieur desdits boîtiers précédents (22, 23).

7. Dispositif de stimulation physiologique magnétique selon la revendication 6, caractérisé en ce que le boîtier contenant l'unité d'élément rotatif (A) est constitué par un évidement (22) ménagé dans des lunettes (21).

8. Dispositif de stimulation physiologique magnétique selon l'une quelconque des revendications 1 à 7, caractérisé en ce que l'unité de source d'énergie électrique (6) produit des impulsions de tension constantes ou suivant un cycle périodique.
